# FASCICULE DE BREVET EUROPEEN

(11) **EP 2 782 853 B1**
(45) Date de publication et mention de la délivrance du brevet: **27.07.2016**
(21) Numéro de dépôt: 12794383.5
(22) Date de dépôt: 26.10.2012
(51) Int. Cl.: B65G 27/02, B01J 8/16

(54) **DISPOSITIF DE TRANSPORT VIBRANT HELICOÏDAL**
SPIRALFÖRMIGE VIBRIERENDE TRANSPORTVORRICHTUNG
HELICAL VIBRATING TRANSPORT DEVICE

(30) Priorité: 24.11.2011 FR 1160726
(43) Date de publication de la demande: 01.10.2014
(73) Titulaire: Technical Alliance, 26270 Cliousclat (FR)
(72) Inventeur: MITZKAT, Martin, F-26270 Cliousclat (FR)
(74) Mandataire: Domange, Maxime
(86) Numéro de dépôt international: PCT/FR2012/052469
(87) Numéro de publication internationale: WO 2013/076397

(56) Documents cités:
- US-A- 3 053 380
- US-A- 4 274 953

## Description

La présente invention concerne un dispositif de transport par vibration de produit à traiter, notamment de produit à traiter thermiquement, le long d'une rampe ou goulotte hélicoïdale, notamment sous forme d'une conduite hélicoïdale ou serpent tubulaire, fixée et enroulée autour ou à l'intérieur d'un fût central, apte à être vibré par un module de vibration dont il est solidaire.

Plus particulièrement, la présente invention concerne un tel dispositif comprenant un nouveau module de vibration, apte à être vibré par des moteurs vibrants, de préférence de type moteurs à balourd.

Des appareils élévateurs vibrants hélicoïdaux de ce type, aussi dénommés élévateurs à secousse, ont été décrits dans FR 2 680 637, FR 2 683 896, FR 2 634 187, FR 2 701 861, FR 2 788 260, FR 2 788 336 et FR 2 712 965.

Dans ces appareils, la vibration du fût central entraîne la vibration de la conduite hélicoïdale, du fait que le fût central permet de rigidifier et de soutenir ladite conduite hélicoïdale. Ladite conduite hélicoïdale comporte au moins deux pas d'hélice. La vibration de la rampe ou conduite hélicoïdale provoque la vibration d'un produit particulaire à traiter circulant en progressant par bonds le long de la rampe ou conduite hélicoïdale du fait desdites vibrations. En général, ledit fût central est disposé verticalement. Cette vibration provoque un déplacement en élévation des particules de produit introduites dans ladite rampe ou conduit hélicoïdal, depuis l'extrémité inférieure de la rampe ou conduit hélicoïdal jusqu'à son extrémité supérieure. Les vibrations du fût font donc avancer le produit en vrac contenu dans la rampe ou tube hélicoïdal.

Ces appareillages peuvent être utilisés pour chauffer et/ou refroidir en continu des produits pulvérulents ou particulaires par contact direct avec un fluide caloporteur, circulant à l'extérieur ou à l'intérieur de ladite conduite hélicoïdale de transport, ou par contact avec la paroi de ladite conduite hélicoïdale chauffée par effet Joule par passage d'un courant électrique dans ladite paroi. La chaleur est transférée par une combinaison complexe de conduction, de convexion et de rayonnement vers le produit circulant à l'intérieur de ladite conduite hélicoïdale. Ainsi, l'appareil permet le traitement thermique en continu de solides divisés à des températures allant jusqu'à 700 à 1 000°C, avec des débits pouvant atteindre 15 t/h. L'espace, autour du produit à l'intérieur de la conduite hélicoïdale, peut être occupé par un gaz inerte, un dit fluide caloporteur, un gaz réactif et/ou de l'air de séchage. L'appareillage peut également être utilisé dans des applications sous vide ou en pression continu à l'intérieur de ladite conduite hélicoïdale de transport.

Des élévateurs vibrants de ce type sont commercialisés par la société REVTECH (France) ainsi que par d'autres sociétés telles que la société SINEX (France).

De façon connue, plusieurs critères déterminent les dimensions de ladite conduite hélicoïdale de l'appareillage, à savoir le débit volumétrique, la densité apparente du produit et sa capacité calorifique, les caractéristiques du gaz de traitement ou d'inertage, le temps de séjour, le profil de température recherché et, enfin, la hauteur de déversement souhaité.

L'angle de montée de la rampe ou conduite hélicoïdale, c'est-à-dire l'inclinaison de la spire par rapport à l'horizontale, est compris classiquement entre 1 et 10°, de préférence entre 1 et 5°. La section transversale de ladite conduite hélicoïdale est, de préférence, sensiblement circulaire.

Le déplacement des particules de produit à l'intérieur de la conduite hélicoïdale se fait par petits bonds, la vitesse de transport dudit produit pouvant atteindre 30 cm/s, pour des temps de séjour du produit pouvant durer jusqu'à une heure. Le diamètre de la conduite hélicoïdale sera fonction de la consistance et/ou du flux du produit à traiter, les diamètres étant de 50 à 300 mm. La capacité de traitement, en termes de débit en t/h, de produit à traiter dépend des dimensions et du poids de l'appareil. Le facteur limitant est constitué par la capacité dudit module de vibration de faire vibrer l'appareil, compte-tenu de son poids total.

La vibration du fût central et de la conduite hélicoïdale résulte de la vibration d'un module de vibration, comprenant une platine de transfert de vibration fixée au sommet ou à la base d'un socle support, sur la paroi latérale duquel sont fixés les moteurs vibrants, de telle sorte que ledit socle et ladite platine de transfert de vibration soient aptes à être vibrés par lesdits moteurs vibrants qui sont fixés au dit socle.

Parmi les moteurs vibrants, on peut citer de préférence les moteurs à balourd. Les moteurs vibrants à balourd comprennent en général un capot cylindrique fermé, enfermant un dispositif électrique apte à entraîner en rotation une masse interne excentrée dont la force centrifuge et le moment (ou couple de travail) déterminent l'amplitude de la vibration résultante du socle de base et de sa platine de transfert de vibration. La masse interne excentrée est entraînée en rotation par un dispositif électrique. Ledit capot est entouré d'une bride ou équipé d'une patte de fixation permettant sa fixation sur une platine ou flasque, laquelle platine ou flasque peut coopérer en rotation avec une contre-platine ou contre-flasque ou contre-bride solidaire du socle de base, de façon à permettre une inclinaison variable dudit moteur par rapport à l'axe XX' dudit socle.

Dans la technique antérieure citée ci-dessus, le module de vibration comporte un socle de base comportant une table vibrante ou plateforme vibrante servant de support au fût central, autour duquel ou dans lequel ladite conduite hélicoïdale s'étend, le socle de base étant apte à être actionné en vibration par deux moteurs à balourd disposés contre et autour dudit socle de base, lesdits deux moteurs à balourd étant disposés symétriquement avec une même inclinaison α par rapport à l'axe vertical XX' dudit socle de base, correspondant à l'axe vertical XX' dudit fût central qu'il supporte.

Ainsi disposés et fixés sur ledit socle de base, qui constitue une structure rigide de liaison transversale des deux moteurs à balourd diamétralement opposés, l'actionnement simultanée des deux moteurs à balourd en sens inverse de rotation permet de réaliser ainsi des vibrations hélicoïdales dudit socle de base et d'induire ainsi des vibrations de la plateforme supérieure et donc du fût central qu'il supporte, rigidement solidarisés l'un à l'autre.

Plus particulièrement, la vibration est obtenue par la combinaison de deux mouvements de translation dans ladite direction axiale XX' et de rotation par rapport au dit axe XX'. Ces mouvements combinés de translation et de rotation, d'amplitude relativement réduite, en aller et retour successifs, constituent des successions de vissage par actionnement simultané des moteurs et dévissage par arrêt simultané des moteurs ou mouvements en vrille. En fonction de l'inclinaison α de l'axe YY' des moteurs par rapport à l'horizontale, on favorise la composante de mouvement en translation (α=90°) ou au contraire de mouvement en rotation (α=0°). En pratique, l'amplitude de ces mouvements en translation ou en rotation correspond à une distance de pas plus de 3 cm, plus particulièrement, pas plus de 2 cm, dans la direction XX' pour la translation ou en longueur d'arc de cercle pour le mouvement en rotation. D'autre part, les amplitude et vitesse des déplacements par bonds des particules de produit à l'intérieur de la conduite hélicoïdale dépendent également de la nature du mouvement vibratoire. En pratique, on règle l'inclinaison des deux axes YY' des deux moteurs à balourd d'un angle α de 10 à 60°, plus particulièrement de 30 à 45°, par rapport à l'horizontale (P).

Pour obtenir des mouvements d'aller et retour en translation/rotation et créer ainsi cette vibration de vissage dévissage, il est nécessaire que les deux moteurs comportent un balourd tournant en sens inverse et à même vitesse. La poutre transversale de liaison rigide des deux moteurs assure l'auto synchronisation des moteurs, c'est-à-dire l'homogénéisation de leur intensité et de leur vitesse respectives.

En disposant deux moteurs de façon diamétralement opposée et symétriquement inclinée, comme mentionné ci-dessus, avec une structure de liaison transversale entre les deux moteurs rigides, une auto synchronisation des deux moteurs se produit de façon stable du fait de l'annulation mutuelle des deux forces transversales développées par les deux moteurs respectivement sur la poutre transversale de liaison rigide des deux moteurs, soit en compression simultanée en sens opposé, soit en étirement simultané en sens opposé.

La poutre transversale de liaison des deux moteurs assure donc la reprise des forces en compression et étirement générés par les forces centrifuges ou centripètes symétriques à l'intérieur desdits moteurs, d'où il résulte que la totalité des mouvements est répercutée en mouvement vibratoire de translation/rotation. La poutre transversale de liaison des deux moteurs à balourd assure, en outre, que les deux moteurs sont bien positionnés symétriquement par rapport à un même plan horizontal, de manière à éviter tout couple de torsion.

Des moteurs de ce type sont, notamment, commercialisés par les sociétés FRIEDRICH SCHWINGTECHNIK GmbH (Allemagne), VISAM (Italie) filiale de WAMGROUP, OLI (France) et ITALVIBRAS (Italie). Ces moteurs, de plus forte capacité, présentent une force centrifuge de 180 000 N et un couple de travail ("working moment") de 6 000 kg.cm. Un moteur de ce type, présentant une fréquence de 740 tr/min (12,5 Hz), comprend 8 pôles (4 paires de pôles) et commercialisé par la société FRIEDRICH SCHWINGTECHNIK GmbH sous la référence F6000-8-10.0.

Deux moteurs à balourd de puissance maximale, tel que décrit ci-dessus, permettent d'obtenir une accélération jusqu'à 4 g d'un appareil élévateur vibrant incluant le module de vibration d'un poids de 9 000 kg. Cette accélération de 4 g peut permettre d'atteindre une vitesse de déplacement de particules jusqu'à 0,4 m/s dans une dite conduite hélicoïdale de 8" de diamètre et entourée autour d'un fût de 2,2 m de diamètre sur 15 spires, représentant une hauteur de conduite de 8 m, permettant ainsi de traiter un débit de produit de 10 t/h (tonnes/heure).

Le but de la présente invention est de pouvoir augmenter le poids de l'appareillage, et donc la dimension du fût et de la conduite hélicoïdale, et donc augmenter le débit de produit à traiter jusqu'à au moins 20 t/h, et la vitesse de déplacement des particules au-delà de 0,4 m/s, ce qui requiert en pratique un appareillage d'au moins 15 t pour, par exemple, une conduite de 10 à 18" de diamètre (environ 27 à 49 cm) s'étendant sur au moins 17 spires, entourée autour d'un fût d'au moins 3 m de diamètre, représentant une hauteur de conduite hélicoïdale d'au moins 17 m.

En pratique, dans un appareil élévateur vibrant selon l'invention, on cherche à maximiser les valeurs d'amplitude du mouvement de vibration et, notamment, à réduire la fréquence de vibration, en pratique pas plus de 15 à 20 Hz, plus particulièrement de l'ordre de 10 Hz, pour des amplitudes de mouvement de 2 à 3 cm.

Avec les moteurs à balourd de puissance maximale disponibles sur le marché, il n'est pas possible d'obtenir des vibrations de 10 à 20 Hz de fréquence avec une accélération de 2 à 5 g et des amplitudes de 10 à 30 mm, d'une masse totale d'appareil élévateur vibrant de 15 000 kg requise pour obtenir des débits de traitement de produit de 20 t/h comme mentionné ci-dessus.

Le but de la présente invention est donc de fournir un module de vibration pour élévateur hélicoïdal amélioré, permettant d'entraîner en vibration de plus grande masse et plus grande dimension pour fournir un débit de traitement de produit plus important qu'avec les systèmes de vibration de la technique actuelle impliquant seulement deux moteurs à balourd.

Selon la présente invention, on fournit un nouveau module de vibration comportant une pluralité de paires de moteurs vibrants.

Plus particulièrement, selon la présente invention, on fournit un dispositif de transport vibrant comportant :
- un module de transport comprenant un premier support cylindrique s'étendant dans une direction longitudinale verticale XX', dénommée premier axe, ledit premier support cylindrique supportant une goulotte ou conduite hélicoïdale de même premier axe XX', et
- un module de vibration comprenant un deuxième support de même premier axe XX', comprenant deux platines supérieure et inférieure, dont une platine de transfert de vibrations, au moins, fixée à une extrémité longitudinale dudit premier support cylindrique de manière à permettre de lui transférer lesdites vibrations, ledit deuxième support supportant, au moins, n paires de moteurs vibrants répartis régulièrement sur la périphérie latérale dudit deuxième support selon un même plan horizontal P, de préférence un plan médian horizontal P, de préférence encore sur une paroi périphérique latérale 6 dudit deuxième support, chaque moteur s'étendant dans une direction longitudinale (YiYi' avec i=1 à 2n) selon une même inclinaison α par rapport à l'horizontale, les deux moteurs de chaque paire étant disposés de façon diamétralement opposés à une même distance dudit axe vertical XX', ledit deuxième support comprenant une pièce de liaison rigide entre les différents moteurs vibrants et ladite table de transfert de vibrations telle que l'actionnement simultané en vibration de l'ensemble desdits moteurs est apte à engendrer une vibration hélicoïdale du premier support.

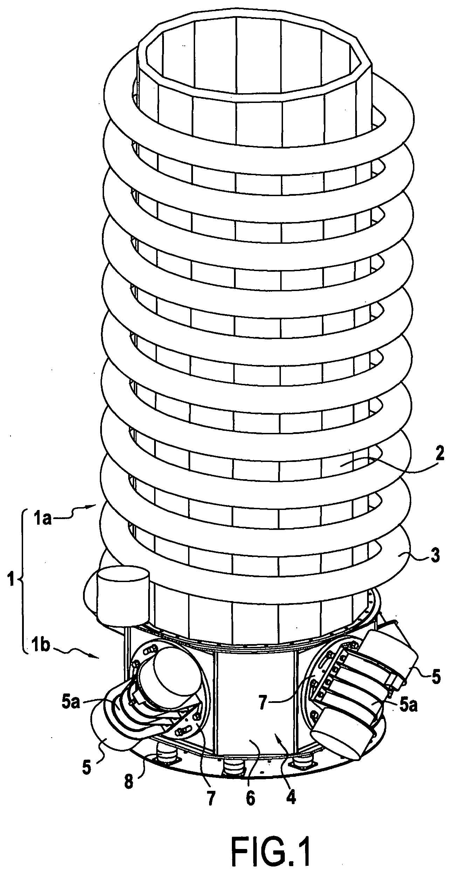

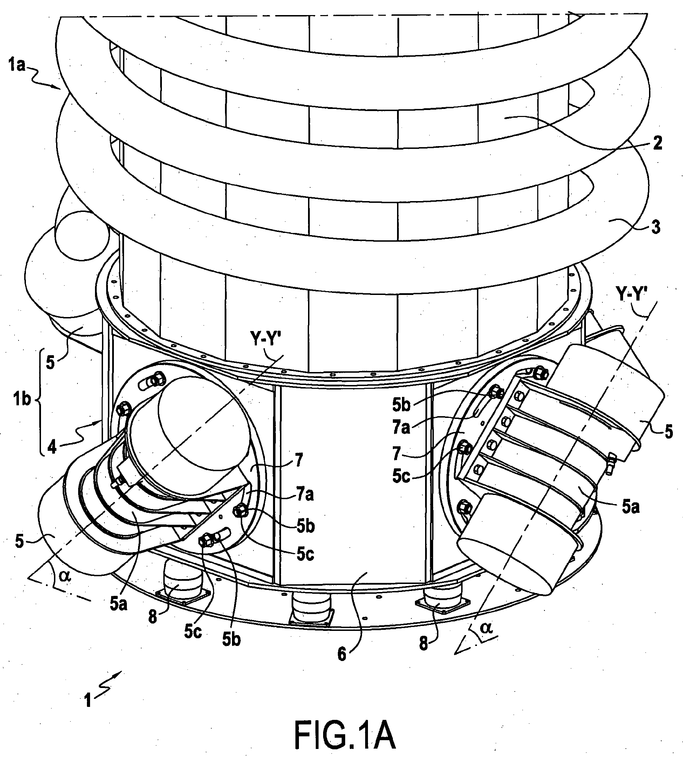

Dans FR 2 223 649, on décrit un transporteur élévateur à rampe hélicoïdale (figure 21) comportant uniquement deux moteurs à balourd diamétralement opposés et de même inclinaison. D'autre part, dans le cas d'un four vibrant à rampe annulaire (figures 1 et 4), on met en oeuvre deux paires de moteurs vibrants disposées en croix, les deux paires de moteurs étant inclinés avec des inclinaisons inversées l'une par rapport à l'autre et étant destinés à être actionnés séparément du fait qu'une première paire de moteurs sert à transporter le produit dans le sens horaire à l'intérieur de la rigole annulaire, tandis que la deuxième paire de moteurs sert à transporter le produit à traiter dans le sens inverse (cf. page 12, lignes 17 à 37).

Dans EP 965805, on décrit un dispositif de séchage dans un récipient en forme de baril, apte à être vibré par deux paires de moteurs vibrants disposées en croix, tous inclinés dans le même sens selon une même inclinaison et actionnés simultanément. Toutefois, du fait du poids de ce four vibrant relativement réduit par rapport à un élévateur hélicoïdal de forte capacité, les deux paires de moteur sont solidarisées par une pièce de liaison formée de deux tubes de liaison disposés en croix. Cette pièce de liaison rigide de tubes disposés en croix ne résisterait pas mécaniquement à la rupture de l'assemblage de tubes ainsi soudés dans le cas de vibrations de forte puissance requises pour faire vibrer un élévateur hélicoïdal de poids et de dimension élevés, conformément au but de la présente invention.

Dans US 3 053 380 on décrit un dispositif de transport vibrant du type décrit ci-dessus selon le préambule de la revendication 1, dans lequel, à la figure 8, il est prévu la mise en oeuvre de deux paires de moteurs à balourd, inclinés dans le même sens. Toutefois, les moteurs sont fixés simplement contre les parois diamétralement opposées d'un socle cubique, sans pièce de liaison transversale entre les parois. Comme représenté sur la figure 9, la portion de tube à section carrée, supportant les moteurs, peut être disposée coaxialement à l'extérieur du dispositif élévateur hélicoïdal et à hauteur intermédiaire entre les extrémités supérieure et, respectivement, inférieure. Il n'y a pas de liaison directe et rigide entre les deux moteurs diamétralement opposés, ce qui risque de provoquer des fissurations. D'autre part, l'auto synchronisation de l'ensemble des moteurs risque de ne pas s'établir à cause de la flexibilité de la liaison.

Selon la présente invention, on fournit un module de vibration comprenant une pièce de liaison rigide, apte à transférer les vibrations générées par l'actionnement simultané d'une pluralité de paires de moteurs vibrants pour faire vibrer un module de transport de forte capacité et résistant mécaniquement, sans risque de rupture de l'assemblage du module de vibration et, en particulier, de ladite pièce de liaison rigide, lorsque celle-ci est mise en vibration pour actionner en secousse en élévateur hélicoïdal de poids et de dimension importants.

Plus précisément, la présente invention fournit un dispositif de transport vibrant hélicoïdal, défini ci-dessus, caractérisé en ce que ladite pièce de liaison rigide est une structure mécano-soudée en partie évidée de même axe vertical XX' comprenant, au moins, un assemblage par soudage d'au moins:
a) une pluralité d'au moins 2n éléments de structure s'étendant dans une direction verticale, dénommés éléments de structure verticaux, comprenant des bords externes diamétralement opposés deux à deux, lesdits éléments de structure verticaux comportant des parties plates s'étendant dans différentes directions radiales ZjZj', avec j= 1 à n, chaque dit élément de structure vertical présentant des sections transversales ouvertes en sections horizontales et sections verticales perpendiculaires à ladite direction radiale, et
b) une pluralité d'au moins 2n éléments de structure s'étendant dans une direction horizontale dénommés éléments de structure horizontaux comprenant des bords externes périphériques diamétralement opposés deux à deux, lesdits éléments de structure horizontaux comportant des parties plates étant situées à différents niveaux dans la direction verticale XX', chaque dit élément de structure horizontal présentant des sections transversales ouvertes en sections verticales, et
c) chaque dit élément de structure vertical étant soudé à au moins un dit élément de structure horizontal et chaque dit élément de structure horizontal étant soudé à au moins un dit élément de structure vertical, au moins 2(n-1) desdits éléments de structure verticaux diamétralement opposés deux à deux comprenant des bords internes soudés à un même élément de liaison verticale, et au moins une partie desdits éléments de structure horizontaux comprenant des bords internes soudés à un même élément de liaison axial verticale, et
d) chaque dit moteur vibrant étant fixé, de préférence par l'intermédiaire d'une première platine de fixation ou premier flasque,
   - soit à au moins un dit bord externe périphérique d'au moins un dit élément de structure vertical et à au moins un dit bord externe périphérique d'au moins un élément de structure horizontal,
   - soit, de préférence, à une partie de paroi latérale soudée à au moins un dit bord externe périphérique d'au moins un dit élément de structure vertical et soudée à au moins un dit bord externe périphérique d'au moins un élément de structure horizontal.

On comprend qu'au moins une partie desdits éléments de structure verticaux et dits éléments de structure horizontaux s'étend en section horizontale avec une continuité de matière depuis ledit élément de liaison axial et leurs bords périphérique externe.

Ce type d'assemblage est avantageux en ce que le soudage des différents éléments constitutif est aisé à réaliser de par la forme de leurs sections transversales mais, surtout, en ce qu'il permet de fournir une pièce de liaison rigide, à la fois de moment d'inertie élevé et de faible poids, de sorte que l'essentiel de la puissance des moteurs vibrants est transféré pour la vibration dudit premier support, la vibration du deuxième support n'en absorbant qu'une quantité relativement réduite.

On entend ici par :
- "plan médian", un plan passant par la mi- longueur dudit moteur dans sa direction longitudinale;
- "vibration hélicoïdale" ou "vibration selon des mouvements hélicoïdaux", des mouvements hélicoïdaux alternés, combinant des déplacements en translation et rotation sur lui-même, successifs, par rapport au dit premier axe vertical XX', en aller lorsque les moteurs sont actionnés et retour lorsque les moteurs sont en arrêts simultanés;
- "actionnement simultané en vibration de l'ensemble desdits moteurs", une succession d'actionnements simultanés et d'arrêts simultanés desdits moteurs;
- "section transversale ", une section dans la direction perpendiculaire à la direction longitudinale, soit verticale pour les premiers profilés horizontaux, soit horizontale pour les éléments de renfort ;
- "bord interne", le bord le plus proche de l'axe vertical XX';
- "section ouverte", une forme ne délimitant pas un espace interne entièrement clos par le contour de la section, contrairement à une section fermée telle que la section d'un tube. Ainsi, des profilés de section ouverte connus sont des poutres dont la section a une des formes de I, T, U ou H;
- "diamétralement opposé", situé au niveau de ladite paroi périphérique, à une même distance dudit axe vertical XX' et dans deux directions alignées opposées à 180° par rapport au dit axe vertical XX', la section horizontale de ladite paroi périphérique s'inscrivant dans un cercle de dit axe XX', notamment, ladite section horizontale de la paroi périphérique formant un polygone régulier;
- "direction radiale", une direction perpendiculaire à la direction axiale verticale XX' et passant par ladite direction axiale XX', et
- i, j, p et n sont des nombres entiers.

De préférence, tous lesdits éléments de structure verticaux comprennent des bords internes soudés à un même élément de liaison verticale axial.

Plus particulièrement, ladite pièce de liaison rigide comprend:
- desdits éléments de structure verticaux entièrement plats, et
- desdits éléments de structure horizontaux entièrement plats.

On comprend que ces éléments de structure entièrement plats constituent des plaques métalliques ou en acier.

Dans une première variante de réalisation, ledit élément de liaison axial est un élément de liaison vertical tubulaire de même axe vertical XX', de préférence tous lesdits éléments de structure verticaux étant soudés à un même dit élément de liaison verticale tubulaire.

Dans une deuxième variante de réalisation, deux dits éléments de structure verticaux, présentant des bords périphériques diamétralement opposés, sont constitués par une même pièce présentant une partie plate centrale verticale s'étendant continument et symétriquement par rapport au dit axe vertical central XX' et formant un dit élément de liaison axial vertical, et les autres élément de structure verticaux présentent des bords internes soudés sur une face de ladite partie plate centrale dudit élément de liaison axial.

Plus particulièrement, dans la première variante de réalisation, ladite pièce de liaison rigide comprend:
- desdits éléments de structure verticaux entièrement plats, s'étendant verticalement entre lesdites platines supérieure et inférieure et radialement depuis sensiblement ledit axe vertical XX' et la périphérie dudit module, et
- desdits éléments de structure horizontaux entièrement plats comprenant deux bords latéraux s'étendant radialement formant desdits bords internes soudés à desdits éléments de structures verticaux, chaque dit élément de structure vertical plat étant soudé à une pluralité de dits éléments de structure horizontaux plats à différents niveaux p dans la direction verticale.

On comprend que chaque élément de structure horizontal occupe un secteur angulaire entre deux éléments de structure verticaux.

Dans les deux modes de réalisation, à chacun des p niveaux dans la direction verticale, 2n dits éléments de structures horizontaux plats sont situés à un même niveau dans la direction verticale.

Plus particulièrement, dans les deux modes de réalisation, ladite pièce de liaison rigide comprend desdits éléments de structure horizontaux à p niveaux dans la direction verticale comportant des parties plates présentant des perforations alignées verticalement dans lesquels se trouve placée une échelle de visites ou aptes à permettre d'y placer une échelle de visite, avec de préférence p=2 à 5.

Plus particulièrement encore, dans les différents modes de réalisation, ladite pièce de liaison rigide comprend :
- des éléments de renfort verticaux constitués de préférence de profilés à sections transversales ouvertes, s'étendant entre deux dites platines supérieure et inférieure ou entre deux parties plates de deux dits éléments de structure horizontaux, et/ou
- des éléments de renfort horizontaux, dont un bord interne radial est soudé à une partie plate d'un dit élément de structure verticale depuis son bord externe périphérique, de préférence deux dits éléments de renfort horizontaux étant soudés sur les deux faces opposées de ladite partie plate.

Lesdits éléments de renfort horizontaux constituent des ailerons latéraux horizontaux complémentaires de fixation de la paroi latérale en vis à vis des platines de fixation desdits moteurs.

Plus particulièrement encore, lesdites paires de moteurs sont au nombre de n=2 à 6, et les différentes directions radiales ZjZj' des différentes paires de moteurs dans ledit plan médian étant espacées angulairement d'un même angle de 90°(n=2), 60° (n=3), 45° (n=4), 36° (n=5), 30° (n=6), de préférence n=2.

Plus particulièrement encore, lesdits moteurs vibrants sont des moteurs à balourd actionnables électriquement en vibration, et lesdits moteurs comprennent des capots cylindriques s'étendant dans des directions axiales longitudinales YY' selon une même inclinaison α par rapport au dit plan médian d'un angle α d'inclinaison de même valeur et dans le même sens de rotation dans deux des plans parallèles au dit premier axe XX' disposés à une même distance dudit premier axe, chaque dit moteur comprenant une masse interne excentrée apte à tourner en rotation interne autour de l'axe longitudinale axiale YY' dudit moteur, chaque dit moteur étant fixé au niveau de son capot à une dite paroi latérale par l'intermédiaire d'une première platine de fixation ou premier flasque solidaire de ladite paroi latérale ou d'une deuxième platine ou deuxième flasque, ladite première platine de fixation ou premier flasque étant fixée à une bride ou collier solidaire dudit capot, ladite première platine ou premier flasque étant fixée contre ladite paroi latérale ou dite deuxième platine ou deuxième flasque, ladite première platine ou premier flasque pouvant adopter plusieurs positions de fixation par rotation de ladite première platine ou premier flasque autour d'un même axe radial de façon à permettre une dite inclinaison alpha variable dudit axe longitudinal dudit moteur.

Plus particulièrement, ladite première platine ou premier flasque comprend des orifices s'étendant chacun sur une portion d'arc de cercle, de préférence de même longueur, les différents orifices étant espacés le long d'un même cercle, de préférence régulièrement espacés, des vis ou doigts de fixation solidaires de ladite paroi latérale ou dite deuxième platine ou deuxième flasque traversant lesdits orifices, la position de ladite première platine ou premier flasque étant ainsi réglable en rotation par rapport à ladite paroi latérale ou dite deuxième platine ou deuxième flasque selon une position quelconque le long de chaque orifice et par décalage des orifices traversant lesdites visses ou doigts.

Un module de vibration d'un dispositif selon l'invention est apte à faire vibrer un dit premier support cylindrique constitué d'un fût central à paroi latérale cylindrique, de préférence à section polygonale, autour de laquelle et contre laquelle est enroulée et fixée une goulotte constituée d'une conduite hélicoïdale, l'ensemble pesant plus de 10 t, de préférence plus de 15 t, et étant apte à traiter thermiquement un produit particulaire, de préférence pulvérulent, avec un débit de plus de 15 t/h, de préférence plus de 20 t/h.

Plus particulièrement, ladite platine supérieure est fixée à une extrémité inférieure dudit premier support et ladite platine inférieure comprend en sous face des plots d'amortissements de préférence en matériau élastomère.

La présente invention fournit également un procédé de mise en oeuvre d'un dispositif de transport vibrant hélicoïdal selon l'invention, caractérisé en ce que l'on réalise une succession d'actionnements et d'arrêts simultanés de l'ensemble desdits moteurs vibrants pour engendrer desdites vibrations hélicoïdales dudit module de transport.

Plus particulièrement, dans le procédé selon l'invention, lesdits moteurs sont des moteurs à balourd comprenant une masse interne excentré apte à tourner en rotation interne autour de l'axe longitudinale axiale YY' dudit moteur, les deux moteurs des différentes paires diamétralement opposés tournant en rotation interne en sens inverse.

D'autres caractéristiques et avantages de la présente invention apparaitront à la lecture de la description détaillée qui va suivre de deux modes de réalisation, faite en liaison aux figures 1 à 3, dans lesquelles :
- la figure 1 représente un dispositif de transport vibrant 1 comportant un module de vibration 1b, équipé de quatre moteurs à balourd 5 et apte à faire vibrer un module de transport 1a, comprenant un fût central cylindrique 2 à section polygonale, supportant une goulotte ou conduite hélicoïdale 3,
- la figure 2 représente une première variante de réalisation d'une pièce de liaison rigide et dit deuxième support 4, un module de vibration 1b vu en perspective,
- la figure 2A est une vue en perspective du module de vibration 1b de la figure 2, sans la platine supérieure 4a,
- la figure 2B est une vue du module de vibration 1b de la figure 2, coupé verticalement au niveau d'un élément de structure vertical 4c4,
- la figure 2C est une vue d'un module de vibration 1b de la figure 2, coupé horizontalement entre deux niveaux, contenant des éléments de structure horizontaux, et
- la figure 3 est une vue en perspective d'un deuxième mode de réalisation d'une pièce de liaison rigide et dit deuxième support 4 d'un module de vibration 1b.

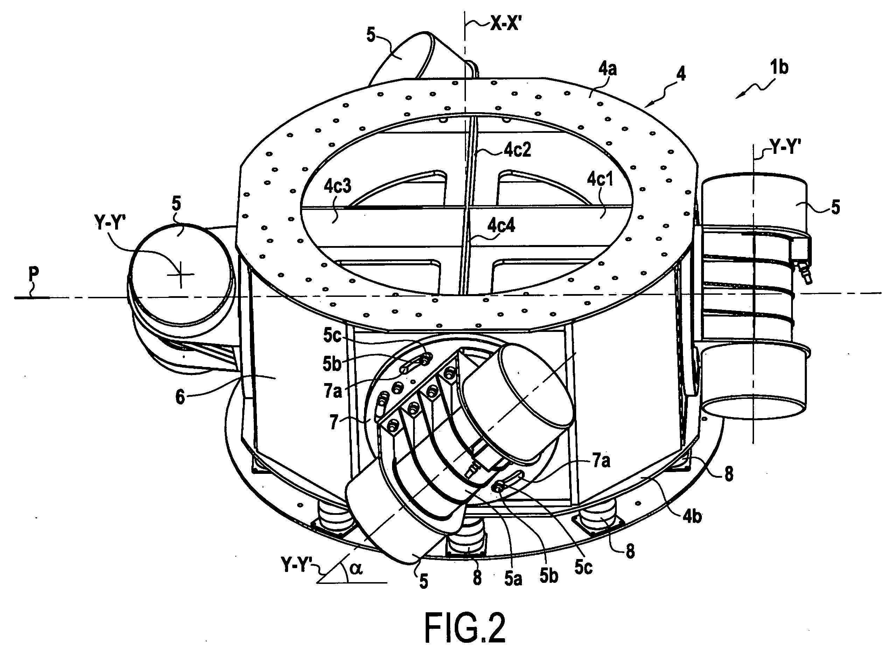

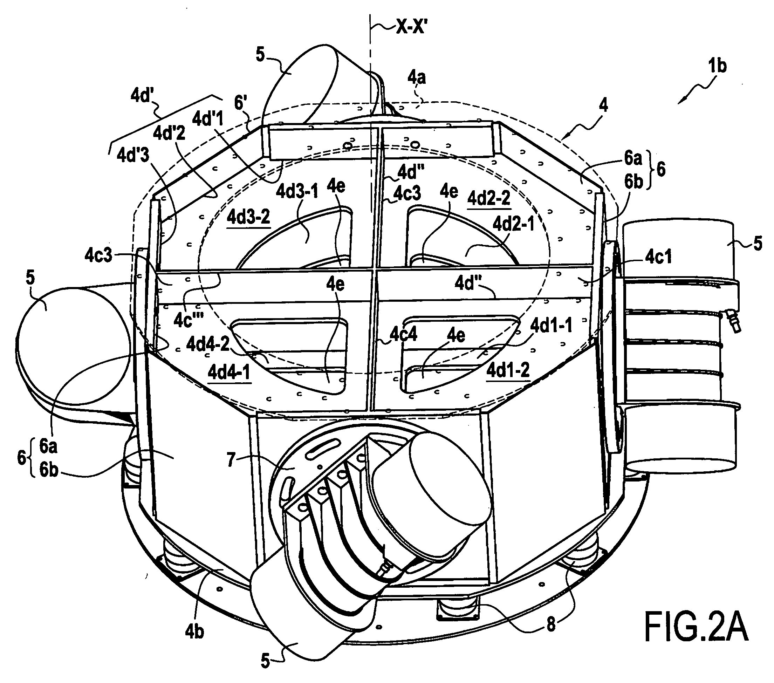

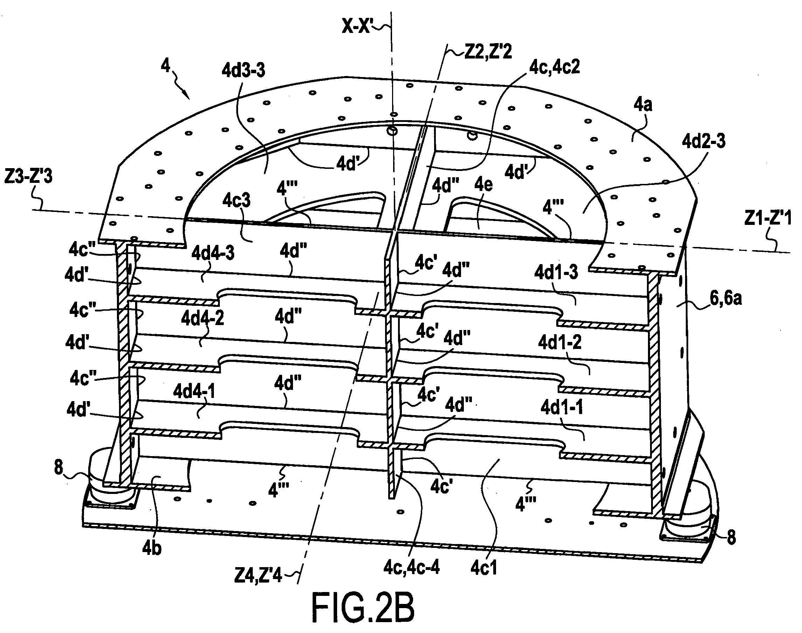

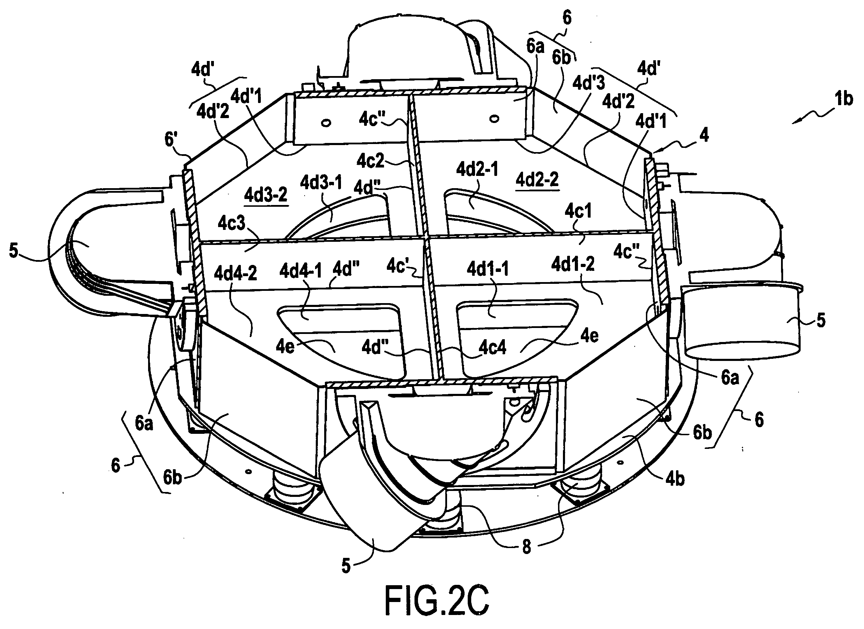

Sur les figures 1 à 3, on a décrit une variante préférée de réalisation d'un module de vibration 1b, comprenant un dit deuxième support 4 d'axe vertical XX', équipé de quatre moteurs à balourds 5.

Ce module de vibration 4 comprend une platine inférieure 4b, de forme annulaire plate, supportant une pièce de liaison rigide constituée d'une structure mécano soudée en partie évidée, de même axe vertical XX', comprenant un assemblage par soudage de quatre éléments de structure verticaux 4c1, 4c2, 4c3, 4c4, constitués de tôles planes, disposés verticalement et radialement depuis l'axe XX'. Plus précisément deux éléments de structure verticaux 4c2 et 4c4 forment une même plaque s'étendant continument et symétriquement par rapport au dit axe vertical central XX' et présentant des bords externes périphériques 4c" diamétralement opposés. Deux autres éléments de structure verticaux 4c1 et 4c3, disposés perpendiculairement à un élément de liaison axial vertical 4c constitué ici par les deux éléments de structure verticaux 4c2 et 4c4. Ces deux éléments 4c1 et 4c3 présentent des bords internes 4c' soudés sur, respectivement, les deux faces de ladite plaque ou élément de liaison axial vertical 4c, au niveau de l'axe vertical central XX'. Chaque élément de structure vertical 4c1 à 4c4 s'étend dans une direction radiale Z1Z'1, Z2Z'2, Z3Z'3 et, respectivement, Z4Z'4, les quatre directions et les quatre éléments 4c1 à 4c4 étant disposés en croix.

Par-dessus les éléments 4c1 à 4c4, est soudée une platine supérieure plate annulaire 4a, de même axe XX'. La platine supérieure 4a repose et est soudée par-dessus les bords supérieurs 6' des panneaux 6a et 6b et bords supérieurs 4c"' des quatre éléments de structure verticaux 4ci, avec i=1 à 4.

Des panneaux de tôle métallique 6a sont disposés verticalement, soudés au niveau des bords externes périphériques 4c" de chacun desdits éléments de structure verticaux 4c1 à 4c4, et perpendiculairement à chacun desdits éléments de structure verticaux 4c1 à 4c4.

Ladite pièce de liaison rigide du deuxième support 4 comprend en outre des éléments de structure horizontaux 4di-j, avec i=1 à 4 et j=1 à 3, au nombre de douze (4d1-1, 4d1-2, 4d1-3, 4d2-1, 4d2-2, 4d2-3, 4d3-1, 4d3-2, 4d3-3, 4d4-1, 4d4-2, 4d4-3), constitués également de plaques ou tôles planes, perforées en partie centrale, 4e.

Les douze éléments de structure horizontaux 4di-j, avec i=1 à 4 et j=1 à 3, sont disposés horizontalement sur trois niveaux, régulièrement répartis sur la hauteur des éléments de structure verticaux 4ci, avec i=1 à 4, de telle sorte que, sur chaque niveau, on dispose quatre éléments de structure horizontaux, chaque élément de structure horizontal étant disposé et soudé entre et, respectivement, contre deux éléments de structure verticaux successifs, c'est-à-dire disposés à 90°. Ainsi, à l'étage du bas, les quatre éléments de structure horizontaux 4d1-1, 4d2-1, 4d3-1 et 4d4-1, sont disposés et soudés, respectivement, de la manière suivante :
- les quatre éléments 4d1-j, avec j=1 à 3, disposés les uns au-dessus des autres, régulièrement répartis sur la hauteur, sont disposés entre les deux éléments verticaux 4c4 et 4c1, et
- les trois éléments 4d2-j, avec j=1 à 3, sont disposés entre les éléments verticaux 4c1 et 4c2, et
- les trois éléments 4d3-j, avec j=1 à 3, sont disposés entre les éléments verticaux 4c2 et 4c3, et
- les trois éléments horizontaux 4d4-j, avec j=1 à 3, sont disposés entre les éléments verticaux 4c3 et 4c4.

Chaque élément de structure horizontal 4di-j comporte deux bords internes latéraux 4d", s'étendant radialement et soudés contre deux plaques verticales 4ci sur toute leur longueur. Chaque élément de structure horizontal 4di-j occupe tout l'espace entre deux éléments de structure verticaux 4ci disposés à 90°. En conséquence, les deux bords latéraux internes 4d" de chaque élément de structure horizontal 4di-j sont à 90°.

Le contour de l'ensemble des quatre éléments de structure horizontaux 4di-j, avec i=1 à 4, pour chacun des niveaux j=1 à 3, forme en coupe horizontale un octogone correspondant aussi à la forme en coupe des quatre panneaux 6a disposés contre les bords externes verticaux 4c" des quatre éléments de structure verticaux 4ci, avec i=1 à 4, et quatre plaques 6b disposées contre les bords périphériques externes 4d' des éléments 4di-j intercalés entre les plaques 6a.

D'autre part, le périmètre en coupe horizontale des bords externes 4d' et de l'ensemble de la paroi d'enveloppe 6 formé par les panneaux 6a et 6b, présente une forme d'octogone régulier, de sorte que le bord externe périphérique 4d' de chaque élément de structure horizontal 4di-j présente trois sections rectilignes 4d'1, 4d'2 et 4d'3. Les deux sections, à chaque extrémité, 4d'1 et 4d'3, s'étendant depuis les extrémités de chacun des deux éléments de structure verticaux 4ci, entre lesquelles est disposé et soudé ledit élément de structure horizontal, sont de même longueur et disposés à 90°. Et, la section médiane 4d'2, rejoignant les deux extrémités des deux sections terminales 4d'1 et 4d'3, est disposée à 135° par rapport aux sections terminales. On comprend donc que chaque section terminale 4d'1 et 4d'3 s'étend sur la moitié de la largeur d'une plaque verticale 6a supportant un moteur 5. La section médiane 4d'2 présente une longueur double des sections terminales 4d'1 et 4d'3.

Dans une partie centrale de chacun des éléments de structure horizontaux 4ci-j, avec i=1 à 4 et j=1 à 3, se trouve une perforation 4e. Les perforations 4e des trois éléments 4ci-j superposés sont disposées en vis-à-vis les unes par rapport aux autres sur chacun des trois niveaux, alignées verticalement de telle sorte qu'une échelle puisse être disposée à l'intérieur du module de vibration 1b pour permettre à un personnel de maintenance de pouvoir intervenir en tant que de besoin à l'intérieur du module de vibration 1b.

Les éléments de structure verticaux 4c1 à 4c4, ainsi que les plaques 6a et 6b de la paroi d'enveloppe 6, reposent sur une platine inférieure annulaire plate 4b de même axe XX'.

En sous-face de la platine inférieur 4b, sont disposés des plots d'amortissement en matériau élastomère 8.

Lesdits moteurs à balourds 5 comprennent des capots cylindriques fixés à des premières platines de fixation 7, de forme circulaire, elles-mêmes fixées contre les parois latérales 6a. Chacune des parois latérales 6a supporte une dite première platine de fixation 7 et un dit moteur 5. La fixation du capot du moteur 5 contre les premières platines 7 se fait par le biais d'une bride 5a, entourant chaque capot et comportant une deuxième platine 5b, solidaire par des vis et écrous 5c des premières platine de fixation 7. Les quatre premières platines de fixation 7 sont disposées à une même hauteur, symétriquement et perpendiculairement chacune par rapport à un élément de structure vertical 4ci, avec i=1 à 4. Lesdites premières platines de fixation 7 comprennent des lumières 7a, incurvées circulairement et régulièrement espacées les unes des autres de manière à pouvoir déplacer, en rotation sur elles-mêmes, lesdites premières platines de fixation 7 du fait que des vis ou doigts de fixation 7b, solidaires de la paroi 6a, traversent lesdits orifices 7a et permettent de solidariser et fixer lesdites premières platines de fixation 7 à une position donnée de rotation grâce à des écrous 7c.

Les quatre moteurs 5 comprennent des capots cylindriques s'étendant dans des directions axiales longitudinales YY' selon une même inclinaison αpar rapport à l'horizontale d'un angle d'inclinaison de même valeur et dans le même sens de rotation dans deux plans parallèles au dit premier axe disposés à une même distance dudit premier axe. Chaque dit moteur est fixé au niveau de son capot à une dite paroi latérale par l'intermédiaire d'une première platine de fixation 7 solidaire de ladite paroi latérale 6. Ladite première platine 7 peut adopter plusieurs positions de fixation par rotation sur elle-même de ladite première platine autour d'un même axe horizontal radial de façon à permettre une dite inclinaison αvariable dudit axe longitudinal dudit moteur 5.

L'inclinaison de l'angle α par rapport à l'horizontale (P) a une valeur α=30 à 60°, de préférence α = 45°.

Chaque dit moteur comprend une masse interne excentrée apte à tourner en rotation interne autour de l'axe longitudinale axiale YY' dudit moteur.

La masse interne excentrée, en rotation à l'intérieur dudit capot, génère des vibrations poly-directionnelles dans un plan à angle droit de l'axe YY' du capot, vibrations dont les fréquences sont déterminées par la vitesse de rotation et dont les amplitudes sont proportionnelles au poids de ladite masse. Le moteur couplé au module de vibration 4 transmet ses vibrations au dit module.

Les moteurs mis en oeuvre présentent une force centrifuge de 180 000 N et un couple de travail ("working moment") de 6 000 kg.cm. Un moteur de ce type, présentant une fréquence de 740 tr/min (12,5 Hz), comprend 8 pôles (4 paires de pôles) et commercialisé par la société FRIEDRICH SCHWINGTECHNIK GmbH sous la référence F6000-8-10.0. Les deux moteurs diamétralement opposés des deux paires de moteurs tournent en rotation interne en sens inverse de manière à ce que, lorsque l'on réalise une succession d'actionnements et d'arrêts simultanés de l'ensemble desdits moteurs vibrants, on engendre des vibrations hélicoïdales dudit module de vibration au niveau de sa platine supérieure 4a.

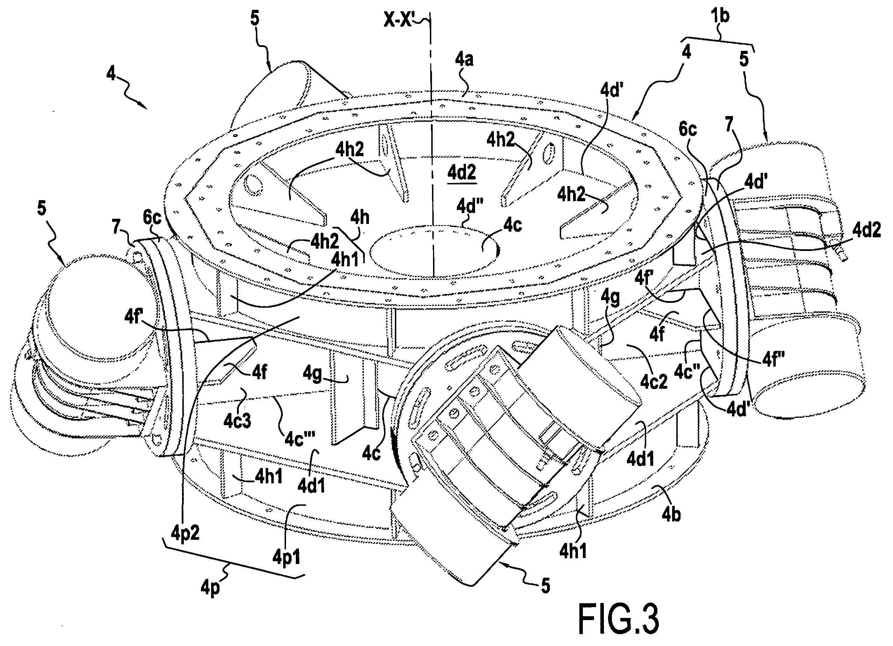

Sur la figure 3, on a représenté une deuxième variante de réalisation d'un module de vibration 4, dont ladite pièce de liaison rigide comporte un élément de liaison axial 4c qui est un élément de liaison vertical tubulaire. Ladite pièce de liaison rigide comporte quatre éléments de structure verticaux 4ci, avec i=1 à 4, constitués de plaques ou tôles métalliques planes verticales disposées radialement, dont un bord interne vertical 4c' est soudé sur la surface externe du tube vertical 4c. Les quatre éléments de structure verticaux 4ci, avec i=1 à 4, sont disposés en croix autour dudit tube vertical central 4c. Les bords externes verticaux 4c" des éléments 4c1 à 4c4 supportent des panneaux de paroi d'enveloppe périphériques 6 (non représentés) ou des flasques circulaires 6c disposés verticalement et perpendiculairement aux dits éléments de structure verticaux 4ci. Chaque flasque 6c supporte directement un dit moteur à balourds 5 ou une dite première platine de fixation 7, sur la face externe de laquelle est fixé un dit moteur à balourd 5. Lesdits flasques 6c ou les panneaux de paroi périphériques 6 (non représentés) sont également supportés par deux éléments de structure horizontaux 4d1, 4d2, disposés et soudés de la manière suivante. Les deux éléments de structure horizontaux 4d1 et 4d2 sont constitués chacun d'une tôle plane horizontale s'étendant depuis la surface externe du tube vertical 4c, sur toute sa circonférence, jusque leur contour périphérique délimitant un octogone. Quatre sections rectilignes dudit octogone forment les parties des bords externes périphériques horizontaux 4d', soudées sur la face interne desdits flasques 6c. Un premier élément de structure horizontal inférieur 4d1 s'étend depuis l'extrémité inférieure du tube de liaison central 4c jusqu'à un dit flasque 6c, et un deuxième élément de structure horizontal supérieur 4d2 s'étend depuis l'extrémité supérieure dudit tube de liaison central 4c jusqu'à un dit flasque 6c.

Le premier élément de structure horizontal inférieur 4d1 est soudé sur les bords inférieurs 4c"' des quatre éléments de structure verticaux 4c1 à 4c4 et le deuxième élément de liaison horizontal supérieur 4d2 est soudé sur les bords supérieurs 4c"' des quatre éléments de structure verticaux 4c1 à 4c4.

Des éléments de renfort horizontaux sont constitués, chacun, par deux ailerons 4f disposés et soudés contre les deux faces opposées de chaque dit élément de structure vertical 4c1 à 4c4. Chaque aileron 4f comprend un bord interne latéral radial horizontal 4f', s'étendant depuis le bord externe périphérique vertical 4c" dudit élément de structure vertical 4ci, et un bord périphérique externe 4f" s'étendant perpendiculairement au dit bord périphérique externe vertical 4c" et soudé sur une face d'un dit flasque 6c. Chaque aileron 4f est situé sensiblement à mi-hauteur dudit élément de structure vertical 4ci. Ainsi, chacun des quatre flasques 6c est supporté par deux ailerons 4f, disposés de part et d'autre de l'élément de structure vertical 4ci supportant ledit flasque et les deux éléments de structure horizontaux inférieur 4d1 et supérieur 4d2.

Les deux éléments de structure horizontaux 4d1 et 4d2 sont reliés en outre par des éléments de renfort verticaux 4g, disposés entre deux éléments de structure verticaux 4ci consécutifs, disposés à 90°. Les quatre éléments de renfort verticaux 4g sont constitués de profilés à section transversale, ouverte, en forme de T.

Ladite pièce rigide mécano soudée comporte en outre deux éléments de liaison tubulaire 4p, disposés respectivement en dessous du premier élément de liaison horizontal inférieur 4d1 et au-dessus du deuxième élément de liaison horizontal 4d2, les deux portions de tube 4p étant disposées selon un même axe XX' que ledit tube central 4c. Le premier élément de liaison tubulaire inférieur 4p1 assure la liaison par soudage de la face inférieure du premier élément de structure horizontal 4d1 et de la face supérieure d'une platine inférieure plate annulaire 4b. Et, le deuxième élément de liaison tubulaire 4p2 assure la liaison par soudage de la face supérieure du deuxième élément de structure horizontal 4d2 et la face inférieure d'une platine supérieure annulaire 4a, lesdites platines, inférieure 4b et supérieure 4a, annulaires, étant de même axe XX'. Des éléments de renfort verticaux 4h, disposés radialement, complètent la rigidification de la liaison entre, d'une part, l'élément de structure horizontal inférieur 4d1 et la platine inférieure 4b, et, d'autre part, entre l'élément de structure horizontal supérieur 4d2 et la platine supérieure 4a. Parmi les éléments de renfort verticaux complémentaires 4h disposés radialement, on distingue des éléments de renfort externes 4h1, soudés, portant un bord interne vertical soudé sur la face externe desdits éléments de liaison tubulaires 4p1 et 4p2, et des éléments de renfort verticaux internes 4h2, soudés sur la face interne desdits éléments de liaison tubulaires 4p1 et 4p2.

Les deux modules de vibration 4 des deux modes de réalisation visés ci-dessus peuvent supporter quatre moteurs à balourd tels que décrits précédemment, tous inclinés d'un même angle α par rapport à un plan horizontal et tous inclinés dans le même sens de rotation, avec deux moteurs diamétralement opposés de chaque paires de moteurs 5 tournant en rotation interne en sens inverse. Ces moteurs 5 sont commercialisés par la société FRIEDRICH SCHWINGTECHNIK GmbH et présentent les caractéristiques décrites ci-dessus. Ainsi, il est possible de faire vibrer un fût 2 supportant une conduite hélicoïdale 3 d'un dispositif de transport vibrant hélicoïdal 1 permettant de traiter jusqu'à 20 t/h de particules pouvant atteindre des vitesses de déplacement de 50 cm/s dans une conduite hélicoïdale de 10 à 18" de diamètre (273 mm à environ 491 mm). L'ensemble du fût central 2 et de la conduite 3 peut peser plus de 15 t pour un fût de 3 m de diamètre et une hauteur de conduite de 17 m s'étendant sur 17 spires, ce qui n'était pas possible de réaliser avec des modules de vibration équipés de seulement deux moteurs à balourd.

## Revendications

1. Dispositif de transport vibrant (1) comportant :
- un module de transport (1a) comprenant un premier support cylindrique (2) s'étendant dans une direction longitudinale verticale (XX'), dénommée premier axe, ledit premier support cylindrique supportant une goulotte ou conduite hélicoïdale (3) de même premier axe (XX'), et
- un module de vibration (1b) comprenant un deuxième support (4) de même premier axe (XX'), comprenant deux platines supérieure et inférieure (4a,4b), dont une platine de transfert de vibrations, au moins, fixée à une extrémité longitudinale dudit premier support cylindrique de manière à permettre de lui transférer lesdites vibrations, ledit deuxième support supportant, au moins, n paires de moteurs vibrants (5) répartis régulièrement sur la périphérie latérale dudit deuxième support selon un même plan horizontal (P), de préférence un plan médian horizontal (P), de préférence encore sur une paroi périphérique latérale (6) dudit deuxième support, chaque moteur s'étendant dans une direction longitudinale (YiYi' avec i=1 à 2n) selon une même inclinaison αpar rapport à l'horizontale, les deux moteurs de chaque paire étant disposés de façon diamétralement opposés, ledit deuxième support comprenant une pièce de liaison rigide entre les différents moteurs vibrants et ladite table de transfert de vibrations telle que l'actionnement simultané en vibration de l'ensemble desdits moteurs est apte à engendrer une vibration hélicoïdale du premier support,
**caractérisé en ce que** ladite pièce de liaison rigide est une structure mécano-soudée en partie évidée de même axe vertical XX' comprenant, au moins, un assemblage par soudage d'au moins:
a) une pluralité d'au moins 2n éléments de structure s'étendant dans une direction verticale, dénommés éléments de structure verticaux (4ci avec i=1 à 2n), comprenant des bords externes (4c') diamétralement opposés deux à deux, lesdits éléments de structure verticaux comportant des parties plates s'étendant dans différentes directions radiales ZjZj', avec j= 1 à n, chaque dit élément de structure vertical présentant des sections transversales ouvertes en sections horizontales et sections verticales perpendiculaires à ladite direction radiale, et
b) une pluralité d'au moins 2n éléments de structure s'étendant dans une direction horizontale dénommés éléments de structure horizontaux (4dj, 4di-j, avec i=1 à 4, j= 1 à p avec p=2 ou 3) comprenant des bords externes périphériques (4d') diamétralement opposés deux à deux, lesdits éléments de structure horizontaux comportant des parties plates étant situées à différents niveaux dans la direction verticale (XX'), chaque dit élément de structure horizontal présentant des sections transversales ouvertes en sections verticales, et
c) chaque dit élément de structure vertical (4ci) étant soudé à au moins un dit élément de structure horizontal (4dj, 4di-j, avec i=1 à 4, j=1 à p avec p=2 ou 3) et chaque dit élément de structure horizontal étant soudé à au moins un dit élément de structure vertical, au moins 2(n-1) desdits éléments de structure verticaux diamétralement opposés deux à deux (4c1, 4c3) comprenant des bords internes (4c') soudés à un même élément de liaison verticale, et au moins une partie desdits éléments de structure horizontaux comprenant des bords internes (4d") soudés à un même élément de liaison axial verticale (4c), et
d) chaque dit moteur vibrant (5) étant fixé, de préférence par l'intermédiaire d'une première platine de fixation ou premier flasque (7),
- soit à au moins un dit bord externe périphérique (4c") d'au moins un dit élément de structure vertical (4ci, avec i=1 à 4) et à au moins un dit bord externe périphérique (4d') d'au moins un élément de structure horizontal,
- soit, à une partie de paroi latérale (6) soudée à au moins un dit bord externe périphérique (4c") d'au moins un dit élément de structure vertical (4ci) et soudée à au moins un dit bord externe périphérique (4d') d'au moins un élément de structure horizontal (4dj, 4di-j).

2. Dispositif selon la revendication 2, **caractérisé en ce que** tous lesdits éléments de structure verticaux comprennent des bords internes (4c') soudés à un même élément de liaison verticale axial (4c).

3. Dispositif selon la revendication 1 ou 2, **caractérisé en ce que** ladite pièce de liaison rigide comprend:
- desdits éléments de structure verticaux entièrement plats (4ci),
et
- desdits éléments de structure horizontaux entièrement plats (4di, 4di-j, 4e, 4e-j).

4. Dispositif selon l'une des revendications 1 à 3, **caractérisé en ce que** ledit élément de liaison axial (4c) est un élément de liaison vertical tubulaire de même axe vertical (XX'), de préférence tous lesdits éléments de structure verticaux (4ci) étant soudés à un même dit élément de liaison verticale tubulaire.

5. Dispositif selon l'une des revendications 1 à 3, **caractérisé en ce que** deux dits éléments de structure verticaux (4c2, 4c4), présentant des bords périphériques diamétralement opposés, sont constitués par une même pièce présentant une partie plate centrale verticale s'étendant continument et symétriquement par rapport au dit axe vertical central (XX') et formant un dit élément de liaison axial vertical (4c), et les autres élément de structure verticaux (4c1, 4c3) présentent des bords internes (4c') soudés sur une face de ladite partie plate centrale dudit élément de liaison axial.

6. Dispositif selon l'une des revendications 1 à 4, **caractérisé en ce que** ladite pièce de liaison rigide comprend:
- desdits éléments de structure verticaux (4ci) entièrement plats, s'étendant verticalement entre lesdites platines supérieure (4a) et inférieure (4b) et radialement depuis sensiblement ledit axe vertical XX' et la périphérie dudit module, et
- desdits éléments de structure horizontaux entièrement plats (4di-j) comprenant deux bords latéraux s'étendant radialement formant desdits bords internes (4d") soudés à desdits éléments de structures verticaux (4ci), chaque dit élément de structure vertical plat étant soudé à une pluralité de dits éléments de structure horizontaux plats à différents niveaux (p) dans la direction verticale.

7. Dispositif selon les revendications 5 et 6, **caractérisé en ce qu'**à chacun des p niveaux dans la direction verticale, 2n dits éléments de structures horizontaux plats (4di-j, avec i=1 à 2n, n=2 et j=1 à 3) sont situés à un même niveau dans la direction verticale.

8. Dispositif selon l'une des revendications 1 à 7, **caractérisé en ce que** ladite pièce de liaison rigide comprend desdits éléments de structure horizontaux à p niveaux dans la direction verticale comportant des parties plates présentant des perforations (4e) alignées verticalement dans lesquels se trouve placée une échelle de visites ou aptes à permettre d'y placer une échelle de visite, avec de préférence p=2 à 5.

9. Dispositif selon l'une des revendications 1 à 8, **caractérisé en ce que** ladite pièce de liaison rigide comprend :
- des éléments de renfort verticaux (4g, 4h) constitués de préférence de profilés à sections transversales ouvertes, s'étendant entre deux dites platines supérieure et inférieure (4a, 4b) ou entre deux parties plates de deux dits éléments de structure horizontaux, et/ou
- des éléments de renfort horizontaux (4f), dont un bord interne radial est soudé à une partie plate d'un dit élément de structure verticale depuis son bord externe périphérique (4c"), de préférence deux dits éléments de renfort horizontaux étant soudés sur les deux faces opposées de ladite partie plate.

10. Dispositif selon l'une des revendications 1 à 9 **caractérisé en ce que** lesdites paires de moteurs sont au nombre de n=2 à 6, et les différentes directions radiales (ZjZj') des différentes paires de moteurs dans ledit plan médian étant espacées angulairement d'un même angle béta de 90°(n=2), 60° (n=3), 45° (n=4), 36° (n=5), 30° (n=6).

11. Dispositif selon l'une des revendications 1 à 10, **caractérisé en ce que** lesdits moteurs vibrants (5) sont des moteurs à balourd actionnables électriquement en vibration, et lesdits moteurs comprennent des capots cylindriques s'étendant dans des directions axiales longitudinales (YY') selon une inclinaison d'un angle α de même valeur et dans le même sens de rotation dans des plans parallèles au dit premier axe XX' disposés à une même distance dudit premier axe, chaque dit moteur comprenant une masse interne excentrée apte à tourner en rotation interne autour de l'axe longitudinale axiale YY' dudit moteur, chaque dit moteur étant fixé au niveau de son capot à une dite paroi latérale par l'intermédiaire d'une première platine de fixation ou premier flasque (7) solidaire de ladite paroi latérale (6) ou d'une deuxième platine ou deuxième flasque (6c), ladite première platine de fixation ou premier flasque (7) étant fixée à une bride ou collier (5a) solidaire dudit capot, ladite première platine ou premier flasque (7) étant fixée contre ladite paroi latérale (6) ou dite deuxième platine ou deuxième flasque (6c), ladite première platine ou premier flasque (7) pouvant adopter plusieurs positions de fixation par rotation de ladite première platine ou premier flasque autour d'un même axe radial de façon à permettre une dite inclinaison alpha variable dudit axe longitudinal dudit moteur(5).

12. Dispositif selon la revendication 11, **caractérisé en ce que** ladite première platine ou premier flasque (7) comprend des orifices (7a) s'étendant chacun sur une portion d'arc de cercle, de préférence de même longueur, les différents orifices étant espacés le long d'un même cercle, de préférence régulièrement espacés, des vis ou doigts de fixation (7b) solidaires de ladite paroi latérale (6) ou dite deuxième platine ou deuxième flasque (6c) traversant lesdits orifices (7a), la position de ladite première platine ou premier flasque étant ainsi réglable en rotation par rapport à ladite paroi latérale (6) ou dite deuxième platine ou deuxième flasque (6c) selon une position quelconque le long de chaque orifice et par décalage des orifices (7a) traversant lesdites visses ou doigts (7b).

13. Dispositif selon l'une des revendications 1 à 12, **caractérisé en ce que** ladite platine supérieure est fixée à une extrémité inférieure dudit premier support et ladite platine inférieure comprend en sous face des plots d'amortissements (8) de préférence en matériau élastomère.

14. Procédé de mise en oeuvre d'un dispositif selon l'une des revendications 1 à 13, **caractérisé en ce que** l'on réalise une succession d'actionnements et d'arrêts simultanés de l'ensemble desdits moteurs vibrants pour engendrer desdites vibrations hélicoïdales dudit module de transport.

15. Procédé selon la revendication 14, **caractérisé en ce que**, lesdits moteurs sont des moteurs à balourd (5) comprenant une masse interne excentré apte à tourner en rotation interne autour de l'axe longitudinale axiale YY' dudit moteur, les deux moteurs des différentes paires diamétralement opposés tournant en rotation interne en sens inverse.

## Patentansprüche

1. Vibrationstransportvorrichtung (1), umfassend:
- ein Transportmodul (1a), das einen ersten zylindrischen Träger (2), welcher sich in einer als erste Achse bezeichneten vertikalen Längsrichtung (XX') erstreckt, umfasst, wobei der erste zylindrische Träger eine spiralförmige Rinne oder Leitung (3) mit gleicher erster Achse (XX') trägt, und
- ein Vibrationsmodul (1b), das einen zweiten Träger (4) mit gleicher erster Achse (XX') umfasst, der zwei Platten, eine obere und eine untere (4a, 4b), umfasst, darunter wenigstens eine Schwingungsübertragungsplatte, die an einem Längsende des ersten zylindrischen Trägers befestigt ist, um die Schwingungen auf ihn zu übertragen, wobei der zweite Träger wenigstens n Paare von Vibrationsmotoren (5) trägt, die über den Seitenumfang des zweiten Trägers, in einer gleichen horizontalen Ebene (P), vorzugsweise einer horizontalen Mittelebene (P), weiterhin vorzugsweise an einer Umfangsseitenwand (6) des zweiten Trägers, gleichmäßig verteilt sind, wobei ein jeder Motor sich in einer Längsrichtung (YiYi', mit i = 1 bis 2 n), in einer gleichen Neigung α gegenüber der Horizontalen erstreckt, wobei die beiden Motoren eines jeden Paares diametral gegenüberliegend angeordnet sind, wobei der zweite Träger ein starres Verbindungsteil zwischen den unterschiedlichen Vibrationsmotoren und dem Schwingungsübertragungstisch umfasst, das derart ist, dass die gleichzeitige Schwingungsbetätigung aller Motoren geeignet ist, eine spiralförmige Schwingung des ersten Trägers zu erzeugen,
**dadurch gekennzeichnet, dass** das starre Verbindungsteil eine teilweise ausgesparte, mechanisch-geschweißte Struktur mit gleicher vertikaler Achse XX' ist, umfassend wenigstens eine Schweißanordnung aus wenigstens:
a) einer Vielzahl von wenigstens 2 n Strukturelementen, die sich in einer vertikalen Richtung erstrecken, welche als vertikale Strukturelemente (4ci, mit i = 1 bis 2n) bezeichnet werden, die paarweise diametral gegenüberliegende Außenkanten (4c') umfassen, wobei die vertikalen Strukturelemente flache Teile umfassen, die sich in unterschiedlichen radialen Richtungen ZjZj', mit j = 1 bis n, erstrecken, wobei jedes vertikale Strukturelement offene Querschnitte aus horizontalen Abschnitten und vertikalen Abschnitten, die zu der radialen Richtung senkrecht verlaufen, aufweist, und
b) einer Vielzahl von wenigstens 2 n Strukturelementen, die sich in einer horizontalen Richtung erstrecken, welche als horizontale Strukturelemente (4dj, 4di-j, mit i = 1 bis 4, j = 1 bis p, mit p = 2 oder 3) bezeichnet werden, die paarweise diametral gegenüberliegende äußere Umfangskanten (4d') umfassen, wobei die horizontalen Strukturelemente flache Teile umfassen, die in vertikaler Richtung (XX') in unterschiedlichen Ebenen gelegen sind, wobei jedes horizontale Strukturelement offene Querschnitte aus vertikalen Abschnitten aufweist, und
c) wobei jedes vertikale Strukturelement (4ci) an wenigstens ein horizontales Strukturelement (4dj, 4di-j, mit i = 1 bis 4, j = 1 bis p, mit p = 2 oder 3) geschweißt ist, und wobei jedes horizontale Strukturelement an wenigstens ein vertikales Strukturelement geschweißt ist, wobei wenigstens 2(n-1) der paarweise diametral gegenüberliegenden vertikalen Strukturelemente (4c1, 4c3) Innenkanten (4c') umfassen, die an ein gleiches vertikales Verbindungselement geschweißt sind, und wobei wenigstens ein Teil der horizontalen Strukturelemente Innenkanten (4d") umfasst, die an ein gleiches axiales, vertikales Verbindungselement (4c) geschweißt sind, und
d) wobei jeder Vibrationsmotor (5) vorzugsweise mittels einer ersten Befestigungsplatte oder eines ersten Flansches (7) befestigt ist
- entweder an wenigstens einer äußeren Umfangskante (4c") wenigstens eines vertikalen Strukturelements (4ci, mit i = 1 bis 4) und an wenigstens einer äußeren Umfangskante (4d') wenigstens eines horizontalen Strukturelements,
- oder an einem Seitenwandteil (6), der an wenigstens eine äußere Umfangskante (4c") wenigstens eines vertikalen Strukturelements (4ci) geschweißt ist und an wenigstens eine äußere Umfangskante (4d') wenigstens eines horizontalen Strukturelements (4dj, 4di-j) geschweißt ist.

2. Vorrichtung nach Anspruch 2, **dadurch gekennzeichnet, dass** alle vertikalen Strukturelemente Innenkanten (4c'), die an ein gleiches vertikales, axiales Verbindungselement (4c) geschweißt sind, umfassen.

3. Vorrichtung nach Anspruch 1 oder 2, **dadurch gekennzeichnet, dass** das starre Verbindungsteil umfasst:
- vollständig flache, vertikale Strukturelemente (4ci) und
- vollständig flache, horizontale Strukturelemente (4di, 4di-j, 4e, 4e-j).

4. Vorrichtung nach einem der Ansprüche 1 bis 3, **dadurch gekennzeichnet, dass** das axiale Verbindungselement (4c) ein vertikales, rohrförmiges Verbindungselement mit gleicher vertikaler Achse (XX') ist, wobei vorzugsweise alle vertikalen Strukturelemente (4ci) an ein gleiches vertikales, rohrförmiges Verbindungselement geschweißt sind.

5. Vorrichtung nach einem der Ansprüche 1 bis 3, **dadurch gekennzeichnet, dass** zwei vertikale Strukturelemente (4c2, 4c4), die diametral gegenüberliegende Umfangskanten aufweisen, durch ein gleiches Teil gebildet sind, das einen flachen, mittleren, vertikalen Teil, welcher durchgehend und in Bezug auf die vertikale Mittelachse (XX') symmetrisch verläuft und ein axiales, vertikales Verbindungselement (4c) bildet, aufweist, und die anderen vertikalen Strukturelemente (4c1, 4c3) Innenkanten (4c'), welche an eine Seite des flachen, mittleren Teils des axialen Verbindungselements geschweißt sind, aufweisen.

6. Vorrichtung nach einem der Ansprüche 1 bis 4, **dadurch gekennzeichnet, dass** das starre Verbindungsteil umfasst:
- vollständig flache, vertikale Strukturelemente (4ci), die sich zwischen der oberen Platte (4a) und der unteren Platte (4b) vertikal und von im Wesentlichen der vertikalen Achse XX' und dem Umfang des Moduls aus radial erstrecken, und
- vollständig flache, horizontale Strukturelemente (4di-j), die zwei sich radial erstreckende Seitenkanten, welche an vertikale Strukturelemente (4ci) geschweißte Innenkanten (4d") bilden, umfassen, wobei jedes flache, vertikale Strukturelement an eine Vielzahl von flachen, horizontalen Strukturelementen in unterschiedlichen Ebenen (p) in vertikaler Richtung geschweißt ist.

7. Vorrichtung nach den Ansprüchen 5 und 6, **dadurch gekennzeichnet, dass** in jeder der p Ebenen in vertikaler Richtung 2 n flache, horizontale Strukturelemente (4dij, mit i = 1 bis 2 n, n = 2 und j = 1 bis 3) in einer gleichen Ebene in vertikaler Richtung gelegen sind.

8. Vorrichtung nach einem der Ansprüche 1 bis 7, **dadurch gekennzeichnet, dass** das starre Verbindungsteil horizontale Strukturelemente in p Ebenen in vertikaler Richtung umfasst, mit flachen Teilen, die vertikal fluchtende Durchbrechungen (4e) aufweisen, in denen eine Einstiegsleiter angebracht ist oder die geeignet sind, dort das Platzieren einer Einstiegsleiter zu ermöglichen, wobei vorzugsweise p = 2 bis 5.

9. Vorrichtung nach einem der Ansprüche 1 bis 8, **dadurch gekennzeichnet, dass** das starre Verbindungsteil umfasst:
- vertikale Verstärkungselemente (4g, 4h), die vorzugsweise von Profilen mit offenen Querschnitten gebildet sind, die sich zwischen zwei Platten, einer oberen und einer unteren (4a, 4b), oder zwischen zwei flachen Teilen von zwei horizontalen Strukturelementen erstrecken, und/oder
- horizontale Verstärkungselemente (4f), von denen eine radiale Innenkante an einen flachen Teil eines vertikalen Strukturelements von seiner äußeren Umfangskante (4c") aus geschweißt ist, wobei vorzugsweise zwei horizontale Verstärkungselemente an die beiden gegenüberliegenden Seiten des flachen Teils geschweißt sind.

10. Vorrichtung nach einem der Ansprüche 1 bis 9, **dadurch gekennzeichnet, dass** die Anzahl der Paare von Motoren n = 2 bis 6 beträgt, und wobei die unterschiedlichen radialen Richtungen (ZjZj') der verschiedenen Paare von Motoren in der Mittelebene um einen gleichen Winkel beta von 90° (n = 2), 60° (n = 3), 45° (n = 4), 36° (n = 5), 30° (n = 6) winkelmäßig beabstandet sind.

11. Vorrichtung nach einem der Ansprüche 1 bis 10, **dadurch gekennzeichnet, dass** die Vibrationsmotoren (5) auf elektrische Weise schwingend betätigbare Unwuchtmotoren sind und die Motoren zylindrische Gehäuse umfassen, die sich in axialen Längsrichtungen (YY') in einer Neigung um einen Winkel α mit gleichem Wert und in der gleichen Rotationsrichtung in zu der ersten Achse XX' parallelen Ebenen, die in einem gleichen Abstand von der ersten Achse angeordnet sind, erstrecken, wobei ein jeder Motor eine exzentrisch angeordnete innere Masse umfasst, die geeignet ist, sich um die axiale Längsachse YY' des Motors innen rotierend zu drehen, wobei ein jeder Motor im Bereich seines Gehäuses an einer Seitenwand mittels einer ersten Befestigungsplatte oder eines ersten Flansches (7), die/der mit der Seitenwand (6) fest verbunden ist, oder einer zweiten Platte oder eines zweiten Flansches (6c) befestigt ist, wobei die erste Befestigungsplatte oder der erste Flansch (7) an einer Klammer oder Schelle (5a), welche mit dem Gehäuse fest verbunden ist, befestigt ist, wobei die erste Platte oder der erste Flansch (7) an der Seitenwand (6) oder zweiten Platte oder am zweiten Flansch (6c) befestigt ist, wobei die erste Platte oder der erste Flansch (7) mehrere Befestigungspositionen durch Rotation der ersten Platte oder des ersten Flansches um eine gleiche radiale Achse einnehmen kann, um eine veränderliche Neigung alpha der Längsachse des Motors (5) zu ermöglichen.

12. Vorrichtung nach Anspruch 11, **dadurch gekennzeichnet, dass** die erste Platte oder der erste Flansch (7) Öffnungen (7a), die sich jeweils über einen Kreisbogenabschnitt erstrecken, vorzugsweise mit gleicher Länge, umfasst, wobei die verschiedenen Öffnungen entlang eines gleichen Kreises beabstandet, vorzugsweise gleichmäßig beabstandet sind, wobei Befestigungsschrauben oder -finger (7b), welche mit der Seitenwand (6) oder mit der zweiten Platte oder dem zweiten Flansch (6c) fest verbunden sind, die Öffnungen (7a) durchgreifen, wodurch die Position der ersten Platte oder des ersten Flansches gegenüber der Seitenwand (6) oder zweiten Platte oder zweiten Flansch (6c) in einer beliebigen Position entlang einer jeden Öffnung und durch Versetzen der Öffnungen (7a), welche die Schrauben oder Finger (7b) durchqueren, dreheinstellbar ist.

13. Vorrichtung nach einem der Ansprüche 1 bis 12, **dadurch gekennzeichnet, dass** die obere Platte an einem unteren Ende des ersten Trägers befestigt ist und die untere Platte an der Unterseite Dämpfungsklötze (8), vorzugsweise aus Elastomermaterial, umfasst.

14. Verfahren zur Anwendung einer Vorrichtung nach einem der Ansprüche 1 bis 13, **dadurch gekennzeichnet, dass** eine Folge von gleichzeitigen Betätigungen und Stopps aller Vibrationsmotoren durchgeführt wird, um spiralförmige Schwingungen des Transportmoduls zu erzeugen.

15. Verfahren nach Anspruch 14, **dadurch gekennzeichnet, dass** die Motoren Unwuchtmotoren (5) sind, die eine exzentrisch angeordnete innere Masse umfassen, welche geeignet ist, sich um die axiale Längsachse YY' des Motors innen rotierend zu drehen, wobei die beiden diametral gegenüberliegenden Motoren der verschiedenen Paare sich in entgegengesetzter Richtung innen rotierend drehen.

## Claims

1. A vibrating transport device (1) comprising:
- a transport module (1a) comprising a first cylindrical support (2) extending in a vertical longitudinal direction (XX'), so-called first axis, said first cylindrical support supporting a helicoidal chute or conduit (3) of the same first axis (XX'), and
- a vibration module (1b) comprising a second support (4) of the same first axis (XX'), comprising two upper and lower tables (4a, 4b), whereof one vibration transfer table, at least, fixed to a longitudinal end of said first cylindrical support so as to enable it to transfer said vibrations, said second support supporting at least n pairs of vibrating motors (5) distributed uniformly over the lateral periphery of said second support according to a same horizontal plane (P), preferably a median horizontal plane (P), more preferably over a peripheral lateral wall (6) of said second support, each motor extending in a longitudinal direction (YiYi' with i=1 to 2n) according to the same inclination α relative to the horizontal, the two motors of each pair being arranged diametrically opposed, said second support comprising a rigid connecting piece between the different vibrating motors and said vibration transfer table such that simultaneous actuation in vibration of the assembly of said motors is capable of generating helicoidal vibration of the first support,
**characterised in that** said rigid connecting piece is a structure mecano-welded, partly hollowed out, of the same vertical axis XX' comprising, at least, an assembly by welding of at least:
a) a plurality of at least 2n structural elements extending in a vertical direction, so-called vertical structural elements (4ci with i=1 to 2n), comprising external edges (4c') diametrically opposed in pairs, said vertical structural elements comprising flat parts extending in different radial directions ZjZj', with j= 1 to n, each said vertical structural element having transversal cross-sections open in horizontal cross-sections and vertical cross-sections perpendicular to said radial direction, and
b) a plurality of at least 2n structural elements extending in a horizontal direction so-called horizontal structural elements (4dj, 4di-j, with i=1 to 4, j= 1 to p with p=2 or 3) comprising external peripheral edges (4d') diametrically opposed in pairs, said horizontal structural elements comprising flat parts being located at different levels in the vertical direction (XX'), each said horizontal structural element having transversal cross-sections open in vertical cross-sections, and
c) each said vertical structural element (4ci) being welded to at least one said horizontal structural element (4dj, 4di-j, with i=1 to 4, j=1 to p with p=2 or 3) and each said horizontal structural element being welded to at least one said vertical structural element, at least 2(n-1) of said vertical structural elements diametrically opposed in pairs (4c1, 4c3) comprising internal edges (4c') welded to a same vertical connecting element, and at least one part of said horizontal structural elements comprising internal edges (4d") welded to the same vertical axial connecting element (4c), and
d) each said vibrating motor (5) being fixed, preferably by means of a first mounting plate or first flange (7),
- either to at least one said peripheral external edge (4c") of at least one said vertical structural element (4ci, with i=1 to 4) and to at least one said peripheral external edge (4d') of at least one said horizontal structural element,
- or, preferably, to part of a lateral wall (6) welded to at least one said peripheral external edge (4c") of at least one said vertical structural element (4ci) and welded to at least one said peripheral external edge (4d') of at least one said horizontal structural element (4dj, 4di-j).

2. The device according to Claim 2, **characterised in that** all said vertical structural elements comprise internal edges (4c') welded to the same vertical connecting element axial (4c).

3. The device according to Claim 1 or 2, **characterised in that** said rigid connecting piece comprises:
- entirely flat said vertical structural elements (4ci), and
- entirely flat said horizontal structural elements (4di, 4di-j, 4e, 4e-j).

4. The device according to any one of Claims 1 to 3, **characterised in that** said axial connecting element (4c) is a vertical tubular connecting element of the same vertical axis (XX'), preferably all said vertical structural elements (4ci) being welded to a tubular said same vertical connecting element.

5. The device according to any one of Claims 1 to 3, **characterised in that** two said vertical structural elements (4c2, 4c4) having diametrically opposed peripheral edges are constituted by the same piece having a vertical central flat part extending continuously and symmetrically relative to the said vertical central axis (XX') and forming a said vertical axial connecting element (4c), and the other vertical structural elements (4c1, 4c3) have internal edges (4c') welded onto a face of said flat central part of said axial connecting element.

6. The device according to any one of Claims 1 to 4, **characterised in that** said rigid connecting piece comprises:
- said entirely flat vertical structural elements (4ci), extending vertically between said upper (4a) and lower (4b) tables and radially from substantially said vertical axis XX' and the periphery of said module, and
- said entirely flat horizontal structural elements (4di-j) comprising two lateral edges extending radially forming said internal edges (4d") welded to said vertical structural elements (4ci), each said flat vertical structural element being welded to a plurality of said flat horizontal structural elements at different levels (p) in the vertical direction.

7. The device according to Claims 5 and 6, **characterised in that** at each of the p levels in the vertical direction, 2n said flat horizontal structural elements (4di-j, with i=1 to 2n, n=2 and j=1 to 3) are located at the same level in the vertical direction.

8. The device according to any one of Claims 1 to 7, **characterised in that** said rigid connecting piece comprises said horizontal structural elements at p levels in the vertical direction comprising flat parts having perforations (4e) aligned vertically in which is placed an inspection ladder or is capable of enabling an inspection ladder to be placed therein, with preferably p=2 to 5.

9. The device according to any one of Claims 1 to 8, **characterised in that** said rigid connecting piece comprises:
- vertical reinforcing elements (4g, 4h) constituted preferably of profiles with open transversal cross-sections, extending between two said upper and lower tables (4a, 4b) or between two flat parts of two said horizontal structural elements, and/or
- horizontal reinforcing elements (4f), whereof a radial internal edge is welded to a flat part of a said vertical structural element from its peripheral external edge (4c"), preferably two said horizontal reinforcing elements being welded onto the two opposite faces of said flat part.

10. The device according to any one of Claims 1 to 9, **characterised in that** said pairs of motors number n=2 to 6, and the different radial directions (ZjZj') of the different pairs of motors in said median plane being spaced angularly by the same beta angle of 90° (n=2), 60° (n=3), 45° (n=4), 36° (n=5), 30° (n=6).

11. The device according to any one of Claims 1 to 10, **characterised in that** said vibrating motors (5) are unbalance motors actuatable electrically in vibration, and said motors comprise cylindrical hoods extending in longitudinal axial directions (YY') according to an inclination by an angle α of the same value and in the same direction of rotation in planes parallel to the said first axis XX' arranged at the same distance from said first axis, each said motor comprising an eccentric internal mass capable of turning in internal rotation about the longitudinal axial axis YY' of said motor, each said motor being fixed at the level of its hood to said lateral wall by means of a first mounting plate or first flange (7) solid with said lateral wall (6) or a second plate or second flange (6c), said first mounting plate or first flange (7) being fixed to a flange or collar (5a) solid with said hood, said first plate or first flange (7) being fixed against said lateral wall (6) or said second plate or second flange (6c), said first plate or first flange (7) capable of adopting several fastening positions by rotation of said first plate or first flange about the same radial axis so as to enable said variable αinclination of said longitudinal axis of said motor(5).

12. The device according to Claim 11, **characterised in that** said first plate or first flange (7) comprises orifices (7a) each extending over a portion of an arc of a circle, preferably of the same length, the different orifices being spaced along the same circle, preferably uniformly spaced, of fastening screws or fingers (7b) connected to said lateral wall (6) or said second plate or second flange (6c) passing through said orifices (7a), the position of said first plate or first flange being adjustable in rotation relative to said lateral wall (6) or said second plate or second flange (6c) according to any position along each orifice and by offset of the orifices (7a) passing through said screws or fingers (7b).

13. The device according to any one of Claims 1 to 12, **characterised in that** said upper table is fixed to a lower end of said first support and said lower table comprises damping pins (8) preferably made of elastomer material on the underside.

14. A process for executing a device according to any one of Claims 1 to 13, **characterised in that** a succession of simultaneous actuations and stoppages of the assembly of said vibrating motors is carried out to generate said helicoidal vibrations of said transport module.

15. The process according to Claim 14, **characterised in that** said motors are unbalance motors (5) comprising an eccentric internal mass capable of turning in internal rotation about the longitudinal axial axis YY' of said motor, the two motors of the different diametrically opposed pairs turning in internal rotation in the opposite direction.
